# EUROPEAN PATENT APPLICATION

(11) **EP 4 248 844 A2**
(43) Date of publication of application: **27.09.2023**
(21) Application number: 23159191.8
(22) Date of filing: 28.02.2023
(51) Int. Cl.: A61B 5/00

(54) **PRESSURE SENSING FOR PHYSIOLOGICAL MEASUREMENTS**

(30) Priority: 02.03.2022 US 202263315801 P; 06.12.2022 US 202218062309
(71) Applicant: Meta Platforms Technologies, LLC, Menlo Park, CA 94025 (US)
(72) Inventor: CALDERON, Ramiro, Menlo Park, 94025 (US); TANKIEWICZ, Szymon Michal, Menlo Park, 94025 (US); SHAGA, Ravi Krishna, Menlo Park, 94025 (US); CORONA APARICIO, Edwin, Menlo Park, 94025 (US); NIAMIR, Darien, Menlo Park, 94025 (US)
(74) Representative: Murgitroyd & Company

(57) **Abstract**

Disclosed herein are methods, systems, and media for pressure sensing for physiological measurements. In some embodiments, a method comprises: obtaining sensor data from one or more sensors disposed in or on a wrist-worn device worn on a wrist of a user. The method may involve determining, based on the sensor data, a measure of a contact pressure of a bottom portion of the wrist-worn device on the wrist of the user. The method may involve based on the measure of the contact pressure, providing one or more instructions to the user to adjust the wrist-worn device to bring about a change in the contact pressure toward a target contact pressure or a target contact pressure range. The method may involve initiating a physiological measurement of the user using other sensors disposed in or on the wrist-worn device.

## Description

### RELATED APPLICATIONS

This application claims the benefit of U.S. Provisional Application 63/315,801, filed March 2, 2022, which is assigned to the assignee hereof, and incorporated herein in its entirety by reference.

### BACKGROUND TO THE INVENTION

Wrist-worn devices, such as smart watches, fitness trackers, etc. are becoming increasingly common. Wrist-worn devices may perform many functions, including performing physiological measurements, analyzing movement activities, analyzing sleep, etc. Such functions may rely on sensors that are disposed in and/or on a wrist-worn devices. It is important for physiological measurements and analysis to be accurate, for example, for user safety and health. Accordingly, methods, systems, and media for providing accurate physiological measurements on wrist-worn devices is desired.

### SUMMARY OF THE INVENTION

According to the present invention, there is provided a method for utilizing contact pressure measurements of wrist-worn devices comprising: obtaining sensor data from one or more sensors disposed in or on a wrist-worn device worn on a wrist of a user; determining, based on the sensor data, a measure of a contact pressure of a bottom portion of the wrist-worn device on the wrist of the user; based on the measure of the contact pressure, providing one or more instructions to the user to adjust the wrist-worn device to bring about a change in the contact pressure toward a target contact pressure or a target contact pressure range; and initiating a physiological measurement of the user using other sensors disposed in or on the wrist-worn device.

In some examples, identifying the target contact pressure of the wrist-worn device to the wrist of the user is based at least in part on a type of physiological measurement to be performed using another one or more sensors associated with the wrist-worn device.

In some examples, identifying the target contact pressure of the wrist-worn device to the wrist of the user is based at least in part on an activity the user is engaged in at a present time.

In some examples, identifying the target contact pressure of the wrist-worn device to the wrist of the user is based at least in part on one or more of a current time of day or current ambient temperature.

In some examples, identifying the target contact pressure of the wrist-worn device to the wrist of the user is based at least in part on motion sensor data indicating that the user is in motion.

In some examples, providing instructions to the user to adjust the wrist-worn device to achieve the target contact pressure comprises providing instructions to adjust a band of the wrist-worn device by an amount identified based on a difference between the contact pressure determined and the target contact pressure.

According to the present invention, there is further provided a method for utilizing contact pressure measurements of wrist-worn devices comprising: obtaining sensor data from one or more sensors disposed in or on a wrist-worn device worn on a wrist of a user; determining, based on the sensor data, a measure of a contact pressure of a bottom portion of the wrist-worn device on the wrist of the user; identifying a target contact pressure of the wrist-worn device for a physiological measurement to be made using the wrist-worn device; identifying at least one change to be made in connection with performing the physiological measurement based on a difference between the determined measure of the contact pressure and the target contact pressure; and performing the physiological measurement using the at least one change to generate a physiological metric of the user.

In some examples, the at least one change comprises a change to operating characteristics of at least one component of the wrist-worn device used to perform the physiological measurement.

In some examples, the at least one component is a light source, and wherein the change to the operating characteristics comprises a change in current provided to the light source.

In some examples, the at least one change comprises a change to an analysis of sensor data collected while performing the physiological measurement to generate the physiological metric of the user.

In some examples, the change to the analysis of the sensor data comprises integrating the sensor data over a time duration that is different than a standard integration time duration, wherein the time duration is determined based at least in part on the difference between the determined contact pressure and the target contact pressure.

In some examples, the at least one change comprises a change to a manner in which the physiological metric is logged.

In some examples, the at least one change comprises utilizing motion sensor data collected using one or more motion sensors of the wrist-worn device to generate the physiological metric of the user.

According to the present invention, there is further provided a wrist-worn device comprising: a top surface; a bottom surface; one or more force sensors; and a processor configured to: receive sensor data from the one or more force sensors; determine a measure of a contact pressure of the bottom surface of the wrist-worn device to a wrist of a wearer based on the received sensor data; and perform at least one action based on the received sensor data, wherein the at least one action comprises at least one of: (1) causing instructions to be presented to the wearer to adjust the wrist-worn device based at least in part on the determined measure of the contact pressure; or (2) modify a manner in which a physiological measurement is made using the wrist-worn device based at least in part on the determined measure of the contact pressure.

In some examples, at least one force sensor of the one or more force sensors is a load sensor configured to measure a deflection of the bottom surface of the wrist-worn device.

In some examples, at least one force sensor of the one or more force sensors is a strain gauge type sensor configured to measure strain.

In some examples, the strain gauge type sensor is disposed proximate to the bottom surface of the wrist-worn device.

In some examples, the strain gauge type sensor is disposed proximate to a band connected to a portion of the bottom surface of the wrist-worn device.

In some examples, the at least one force sensor comprises three or more force sensors, and wherein the processor is further configured to determine a tilt of a puck of the wrist-worn device on the wrist of the wearer based on the sensor data received from the three or more force sensors, the puck comprising the top surface, the bottom surface, and a volume enclosed between the top surface and the bottom surface.

Optionally, a method for utilizing contact pressure measurements of wrist-worn devices may comprise: obtaining sensor data from one or more sensors disposed in or on a wrist-worn device worn on a wrist of a user; determining, based on the sensor data, a measure of a contact pressure of a bottom portion of the wrist-worn device on the wrist of the user; based on the measure of the contact pressure, generating a ballistocardiogram signal indicative of a cardiac signal of the user; and using the ballistocardiogram signal to determine at least one physiological measurement of the user.

In some examples, the at least one physiological measurement is a heart rate of the user. In some examples, determining the heart rate of the user comprises determining a periodicity of the ballistocardiogram signal.

In some examples, the bottom portion of the wrist-worn device is configured to be proximate to the radial artery or the ulnar artery of the user. In some examples, the at least one physiological measurement comprises the blood pressure of the user, and wherein the measure of the contact pressure is used to calibrate a relative blood pressure measurement obtained using at least one sensor other than the one or more sensors to an absolute blood pressure measurement. In some examples, the at least one sensor other than the one or more sensors comprises a light emitter and a light detector configured to obtain photoplethysmography data. In some examples, the method further comprises identifying one or more biomarkers in the ballistocardiogram signal, wherein determination of the at least one physiological measurement comprises using timing information associated with the one or more biomarkers. In some examples, the timing information associated with the one or more biomarkers is used in conjunction with a photoplethysmography signal to determine the at least one physiological measurement. In some examples, the one or more biomarkers comprise at least one of: a diastolic point; or a dicrotic notch.

In some examples, determining the at least one physiological measurement comprises utilizing the ballistocardiogram signal and a photoplethysmography signal, and wherein the photoplethysmography signal was obtained using a light emitter and a light detector disposed in a device paired with the wrist-worn device. In some examples, the device paired with the wrist-worn device comprises at least one of: smart glasses; or a virtual reality (VR)/augmented reality (AR) headset.

Optionally, a wrist-worn device may comprise: a top surface; a bottom surface; one or more force sensors; and a processor. The processor may be configured to: receive sensor data from the one or more force sensors; determine a measure of contact pressure of the bottom surface of the wrist-worn device to a wrist of a wearer based on the received sensor data; based on the measure of the contact pressure, generate a ballistocardiogram signal indicative of a cardiac signal of the user; and use the ballistocardiogram signal to determine at least one physiological measurement of the user.

In some examples, the at least one physiological measurement is at least one of: a heart rate of the wearer; a blood pressure of the wearer; or an oxygen saturation of the wearer. In some examples, the at least one physiological measurement comprises the blood pressure of the wearer, and wherein the measure of the contact pressure is used to calibrate a relative blood pressure measurement obtained using at least one sensor other than the one or more sensors to an absolute blood pressure measurement. In some examples, the at least one sensor other than the one or more sensors comprises a light emitter and a light detector configured to obtain photoplethysmography data. In some examples, the light emitter and the light detector are disposed in or on a portion of the wrist-worn device. In some examples, the light emitter and the light detector are disposed in or on a portion of a user device paired with the wrist-worn device. In some examples, the user device paired with the wrist-worn device comprises an Augmented Reality (AR) or Virtual Reality (VR) headset, and wherein the processor is configured to communicate with a processor of the ARNR headset.

In some examples, the processor is further configured to identify one or more biomarkers in the ballistocardiogram signal, wherein determination of the at least one physiological measurement comprises using timing information associated with the one or more biomarkers.

### BRIEF DESCRIPTION OF THE DRAWINGS

Illustrative embodiments are described, by way of example only, in detail below with reference to the following figures.
FIG. 1A is a cross-sectional view of a portion of a wrist-worn device including pressure sensors in accordance with the present invention;
FIG. 1B is a side view of a portion of a wrist-worn device including pressure sensors in accordance with the present invention;
FIGS. 1C and 1D are top views of a portion of a wrist-worn device including multiple pressure sensors in accordance with the present invention;
FIG. 1E is a schematic diagram illustrating use of a strain gauge type pressure sensor in accordance with the present invention;
FIG. 1F is a schematic diagram illustrating use of a compression force type pressure sensor in accordance with the present invention;
FIG. 2 is a diagram illustrating effects of band tightness of a wrist-worn device on physiological measurements;
FIG. 3 is a flowchart of an example process for providing instructions to a wearer of a wrist-worn device to adjust contact pressure in accordance with the present invention;
FIG. 4 is a flowchart of an example process for utilizing pressure sensing for closed loop control of physiological measurements in accordance with the present invention;
FIG. 5 is a flowchart of an example process for utilizing pressure measurements for determining at least one physiological measurement;
FIG. 6 is a simplified block diagram of an example of a computing system that may be implemented as part of a mobile device and/or a user device in accordance with the present invention; and
FIG. 7 is a simplified block diagram of an example of a computing system that may be implemented as part of a server in accordance with the present invention.

The figures depict examples of the present invention for purposes of illustration only. One skilled in the art will readily recognize from the following description that alternative examples of the structures and methods illustrated may be employed without departing from the principles, or benefits touted, of this disclosure.

In the appended figures, similar components and/or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

### DETAILED DESCRIPTION OF EXAMPLES OF THE INVENTION

Wrist-worn devices, such as smart watches and fitness trackers, often include sensors that are used to perform physiological measurements. Such physiological measurements may include photoplethysmography (PPG) based measurements (which may include heart rate, blood pressure, oxygen saturation, etc.), motion sensor based measurements (which may include respiration rate, etc.), electromyography (EMG) based measurements (which may include detection of muscle activity associated with the hand, wrist, arm, and/or fingers), body temperature measurements, or the like. The sensors may be disposed in and/or on the wrist-worn device, for example, disposed in and/or proximate to a back cover of the wrist-worn device that is configured to be in contact with a wrist of the wearer, disposed in and/or proximate to a band of the wrist-worn device that is configured to be in contact with the wrist of the wearer, or the like. For example, PPG-based measurements may be made using one or more light sources (e.g., light emitting diodes (LEDs)) and one or more light detectors that are positioned in and/or on a back cover of the wrist-worn device such that light is emitted toward the wrist of the wearer and light reflected from tissue of the wearer is detected by the one or more light detectors. As another example, EMG-based measurements may be made using surface EMG electrodes disposed in and/or on the back cover of the wrist-worn device, in and/or on the band of the wrist-worn device, or the like.

Accuracy of physiological measurements depends on the quality of the sensor data used to perform the physiological measurements. The quality of the sensor data in turn depends on how well-positioned the sensor(s) are and/or a quality of the contact of the sensor(s) to the skin of the wearer. The quality of sensor positioning, when sensors are disposed proximate to a back cover of a wrist-worn device and/or a band of the wrist-worn device, is dependent on a fit of the wrist-worn device. For example, in instances in which the wrist-worn device is too loose, there may be poor or inconsistent contact of the sensors with the skin of the wearer due to slipping of the wrist-worn device on the wearer's wrist. Conversely, a too tight wrist-worn device may also negatively impact the quality of the physiological measurements. For example, a too tight wrist-worn device may cause temperature artifacts in temperature sensor measurements used to determine body temperature. As another example, in instances in which PPG-based measurements are made, a too tight wrist-worn device may occlude arterial blood flow, thereby causing inaccurate measurements of the absorption spectrum of arterial blood, which may lead to inaccurate heart rate, blood pressure, and/or oxygen saturation measurements. Accordingly, the tightness of the wrist-worn device to the wrist of the wearer is a key component of the quality of physiological measurements made using sensors of the wrist-worn device. The tightness of the wrist-worn device to the wrist of the wearer may be correlated with the contact pressure of the back cover of the wrist-worn device to the wrist of the wearer. Accordingly, in some embodiments, a measured contact pressure of the back cover of the wrist-worn device to the wrist of the wearer may be used to approximate the band tightness.

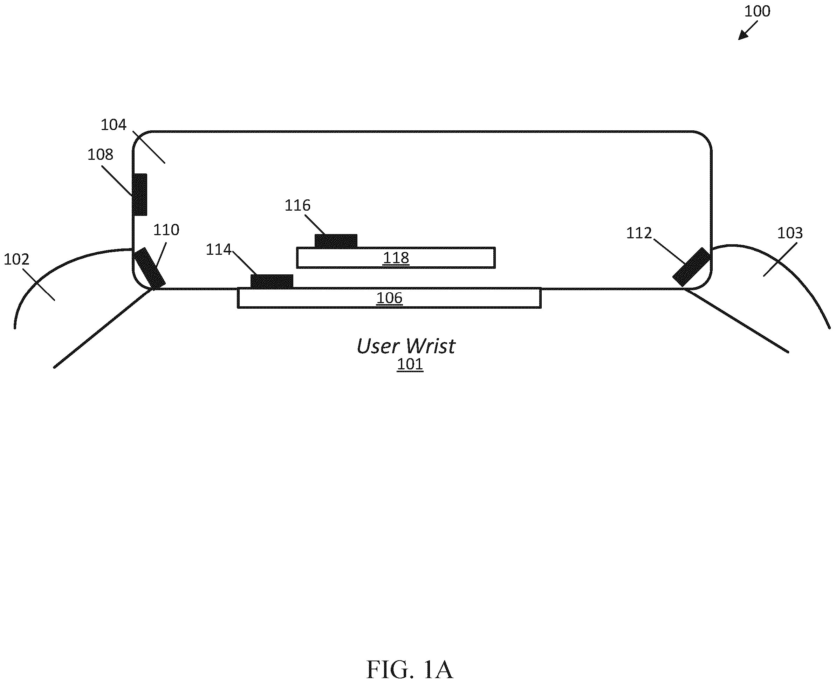

The techniques described herein use pressure sensors disposed in and/or on the wrist-worn device to determine a contact pressure between a back cover of the wrist-worn device to the wrist of the wearer. The pressure sensors may be strain gauge type sensors that detect deflection or bending of a surface the strain gauge type sensor is affixed to, based on the contact pressure and/or compression force type sensors that detect a compression force between two surfaces the compression force type sensor is affixed to. Types and locations of pressure sensors are described in more detail in connection with FIGS. 1A-1F.

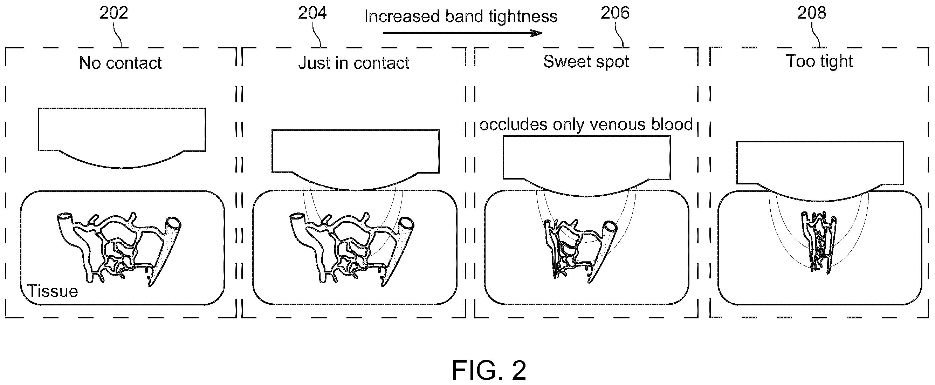

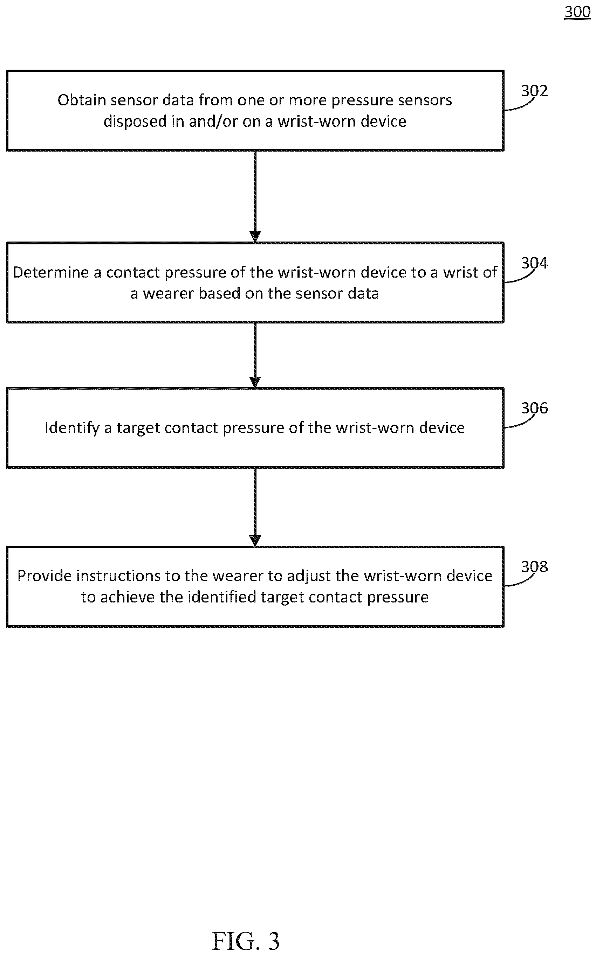

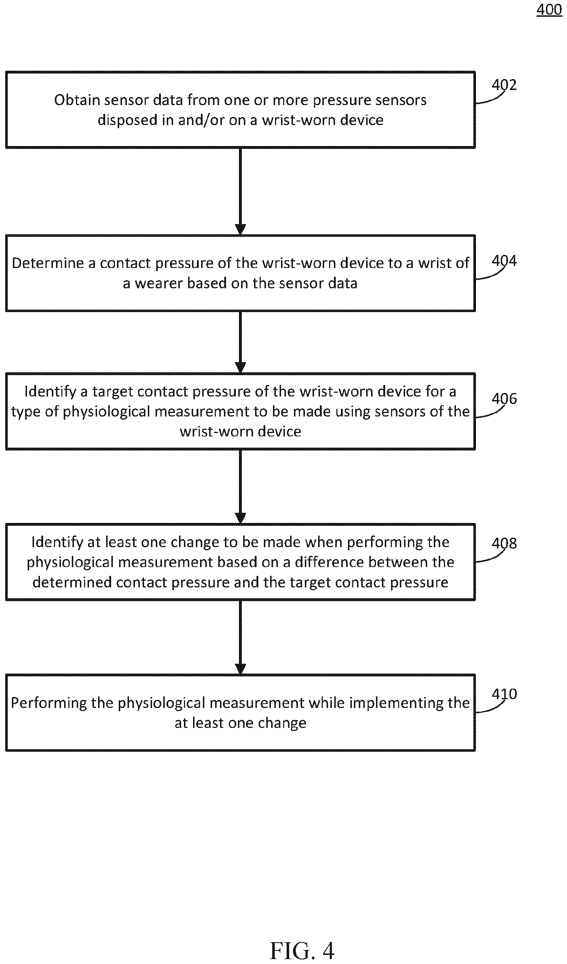

The techniques described herein may identify a target contact pressure. The target contact pressure may be one that is optimal for a particular type of physiological measurement, as shown in and described below in connection with FIG. 2. In some implementations, the target contact pressure may be dependent on a type of physiological measurement to be performed, unique to the individual wearer, based on ambient weather conditions (e.g., temperature, humidity, etc.), based on the current activity state of the wearer (e.g., whether the wearer is exercising, sitting, sleeping, etc.), or any combination thereof. Instructions may be provided to the wearer instructing the wearer to adjust the tightness of the wrist-worn device (and consequently, the contact pressure of the back cover of the device to the wrist of the wearer), e.g., by tightening or loosening the band of the wrist-worn device, as shown in and described below in connection with FIG. 3. Additionally or alternatively, changes may be made to a manner in which physiological measurements are collected based on a difference between the current contact pressure and the target contact pressure to yield more robust and/or accurate physiological measurements. For example, in instances in which PPG-based measurements are performed, an LED current of an LED used to emit light toward the wearer may be changed. As another example, an integration time of sensor data used to make the physiological measurement may be increased to increase signal to noise ratio. As yet another example physiological measurements may be determined which consider other data, such as motion data or body temperature data to ensure a more accurate physiological measurement. Examples of techniques to modify collection of physiological measurements based on contact pressure are shown in and described below in connection with FIG. 4.

A target contact pressure for a particular type of physiological measurement may be an optimal contact pressure at which data collected by sensors to perform the physiological measurement has the highest signal to noise ratio, has relatively low variance between successive measurements, and/or satisfies any other criteria for robustness of the sensor data. The target contact pressure may be a contact pressure where both decreased contact pressure and increased contact pressure yield lower quality sensor data for performing the particular physiological measurement. In other words, the target contact pressure may be a maximum of a function that relates sensor data quality to contact pressure. It should be noted that such a function may vary based on various factors, such as the type of physiological measurement, time of day, wearer activity, ambient weather characteristics, and/or inter-personal variability.

In some examples, optimizing contact pressure of the back cover of the wrist-worn device to the wrist of the wearer (and, consequently, optimizing band tightness) may improve the quality of various physiological measurements. For example, heart rate measurements may be improved by improving sensor contact quality while the wearer is in motion (e.g., exercising, etc.). As another example, oxygen saturation measurements and/or blood pressure measurements may be improved by improving sensor contact quality, by removing venous blood from the field of view of the light emitter(s) and/or light detector(s), etc. As yet another example, skin temperature measurements may be improved by improving a sensor contact quality to the skin of the wearer. As still another example, EMG measurements may be improved by improving a sensor contact quality to the skin of the wearer and/or indicating when EMG electrodes are not in contact with the skin. This may allow for improved detection of false or missed gestures.

In some implementations, a determined contact pressure (e.g., the pressure of the back cover of the wrist-worn device on the wrist of the wearer) may be utilized for purposes other than improving physiological measurements. For example, a determined contact pressure may be used to determine whether or not the wrist-worn device is on the wrist of the wearer at a given time. Determination of whether the wrist-worn device is on the wrist of the wearer may trigger or change various functionalities of the wrist-worn device, such as functions of various buttons or other inputs of the wrist-worn device. As another example, a determined contact pressure may be used to modify antenna power settings of one or more antennas of the wrist-worn device. By way of example, in an instance in which an antenna of the wrist-worn device is disposed proximate to the bottom cover of the wrist-worn device, a transmitting power required by the antenna to adequately transmit signals may depend on a tightness of the band, e.g., due to attenuation of signals transmitted by the antenna from the contact pressure against the wearer's skin. Continuing with this example, in some embodiments, a transmitting power of such an antenna may be modified (e.g., increased or decreased) based at least in part on the contact pressure of the wrist-worn device to the wrist of the wearer.

It should be noted that the term "contact pressure" as used herein generally refers to a measure of force of a portion of a device surface (e.g., a back cover of a wrist-worn device) on an area of body surface (e.g., a wrist of a wearer of the wrist-worn device). As used herein, a "pressure sensor" may include a pressure sensor which measures force per unit area (e.g., pounds per square inch, or the like), or a force sensor which measures a force. In instances in which a force is measured, a measure of contact pressure may be determined based on the measured force, for example, by dividing the measured force by a known surface area (e.g., an area of the bottom cover of the wrist-worn device, or the like).

**FIG. 1A** shows a cross-sectional side view of an example wrist-worn device 100 in accordance with the invention. As illustrated, a back portion (sometimes referred to herein as a "back portion" or a "back cover" portion) rests on a body portion 101 of a body of a wearer (e.g., a wrist surface, an arm surface, or the like). Wrist-worn device 100 includes two band portions 102 and 103, each of which are coupled to an end of a capsule 104 (e.g., via clips, hinges, an adhesive, or the like). A top portion of capsule 104 may include a display screen, and a bottom cover 106 rests on body portion 101. One or more pressure sensors may be affixed to and/or embedded within capsule 104. Example locations of pressure sensors are depicted in FIG. 1A. For example, a pressure sensor 108 is positioned at a side of capsule 104. As another example, pressure sensors 110 and 112 are positioned along a bottom portion of capsule 104, each of pressure sensors 110 and 112 being proximate to an end of capsule 104 at which band portion 102 or band portion 103 is coupled. As yet another example, pressure sensor 114 is positioned along a bottom portion of capsule 104 proximate to bottom cover 106. As still another example, pressure sensor 116 is positioned on a chip or circuit board 118 disposed within capsule 114. In some implementations, circuit board 118 may include one or more sensors suitable for collecting data for performing physiological measurements, such as one or more light-emitting diodes (LEDs), one or more light detectors, one or more accelerometers, one or more gyroscopes, or the like. In some implementations, light from light emitters may shine through bottom cover 106 toward body portion 101, and light reflected from body portion 101 or a region of the body proximate to body portion 101 may be transmitted through bottom cover 106 and captured by one or more light detectors within capsule 104. It should be noted that although five pressure sensors are depicted in FIG. 1A, this is merely exemplary, and, in some implementations, a wrist-worn device may include any suitable number of pressure sensors (e.g., one, two, three, four, six, ten, or the like).

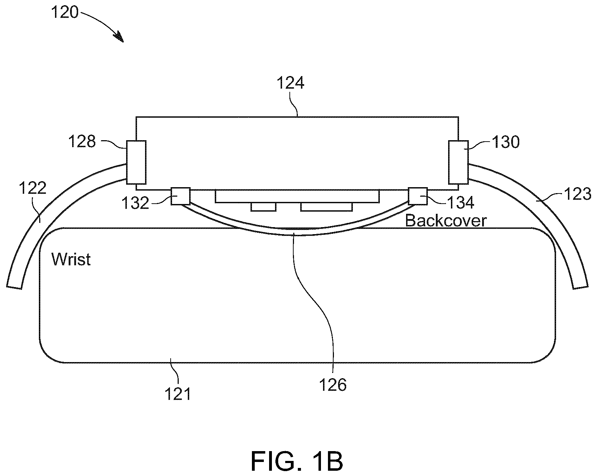

**FIG. 1B** shows a side view of another example wrist-worn device 120. As illustrated, wrist-worn device 120 includes two band portions 122 and 123 coupled to two corresponding ends of a capsule 124. A bottom cover 126 of capsule 124 is configured to rest on a portion of a wrist 121 of a wearer. FIG. 1B illustrates possible locations of pressure sensors that may be included in wrist-worn device 120 configured to sense a pressure of bottom cover 126 to wrist 121. For example, pressure sensors 128 and/or 130 may be positioned on sides of capsule 124 proximate to where band portion 122 or band portion 123 is coupled. As another example, pressure sensors 132 and/or 134 may be positioned proximate to where bottom cover 126 is affixed to a bottom portion of capsule 124. It should be noted that although four pressure sensors are depicted in FIG. 1B, this is merely exemplary, and, in some implementations, any suitable number of pressure sensors other than what is shown in FIG. 1B may be used (e.g., one, two, three, five, six, ten, or the like).

Multiple (e.g., two, three, four, five, six, etc.) pressure sensors may be disposed proximate to a bottom cover of a wrist-worn device such that the pressure sensors are configured to detect variations in pressure across an X-Y plane corresponding to the bottom cover of the wrist-worn device. The variations in pressure across the X-Y plane may be used to detect a tilt of a capsule of the wrist-worn device relative to a body (e.g., a wrist surface) of the wearer. For example, the variations in pressure across the X-Y plane may detect that the capsule is tilted to one side (e.g., due to the wrist-worn device being too big or too small for the wearer). In some implementations, variations in pressure across the X-Y plane may be used to detect where on a top surface (e.g., a display) of the capsule the wearer is pressing or moving a finger. Such pressure variations may be used as user input, for example, on a user interface (e.g., to scroll in a user interface presented on the display, to adjust a volume of audio content being presented by a user device paired to the wrist-worn device, or the like).

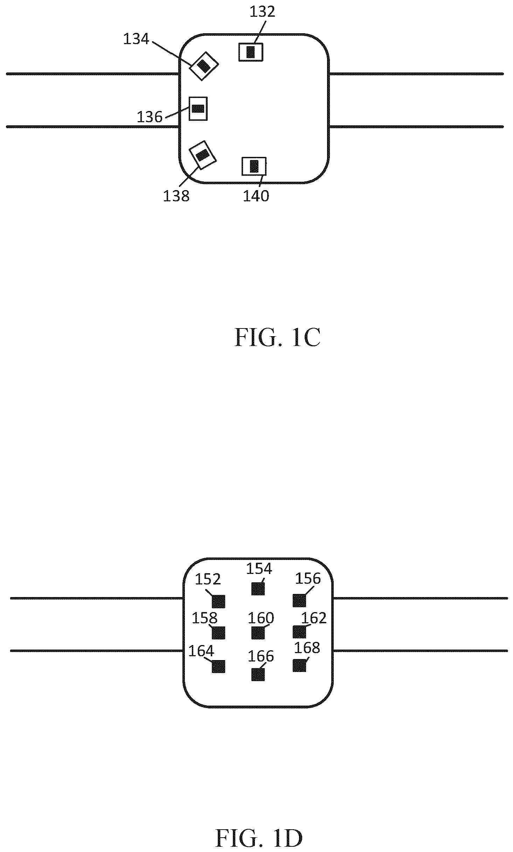

**FIGS. 1C** and **1D** show diagrams of example wrist-worn devices on which multiple pressure sensors have been disposed proximate to the bottom cover. In the example shown in FIG. 1C, pressure sensors 132, 134, 136, 138, and 140 are each affixed to a printed circuit board (PCB), each of which is disposed in a different location proximate to the bottom cover. In the example shown in FIG. 1D, pressure sensors 152, 154, 156, 158, 160, 162, 164, 166, and 168 are disposed on a common PCB which is in turn disposed proximate to the bottom cover. In some implementations, the common PCB may additionally include other sensors and/or one or more processors which may be used for performing physiological measurements, and/or for any other suitable purposes.

In some implementations, a pressure sensor may be a strain gauge type sensor. A strain gauge type sensor may detect bending due to pressure of a bottom cover of the wrist-worn device on a body portion of the wearer. For example, a strain gauge sensor positioned proximate to the bottom cover may detect a bending or a deflection of the bottom cover of the wrist-worn device. As another example, a strain gauge sensor positioned proximate to a side of a capsule of the wrist-worn device may detect a bending or deflection of the side of the capsule, due to, e.g., the band of the wrist-worn device pulling on the side of the capsule. In some examples, a strain gauge type sensor may be affixed to a surface of which the strain gauge type sensor detects deflection or bending.

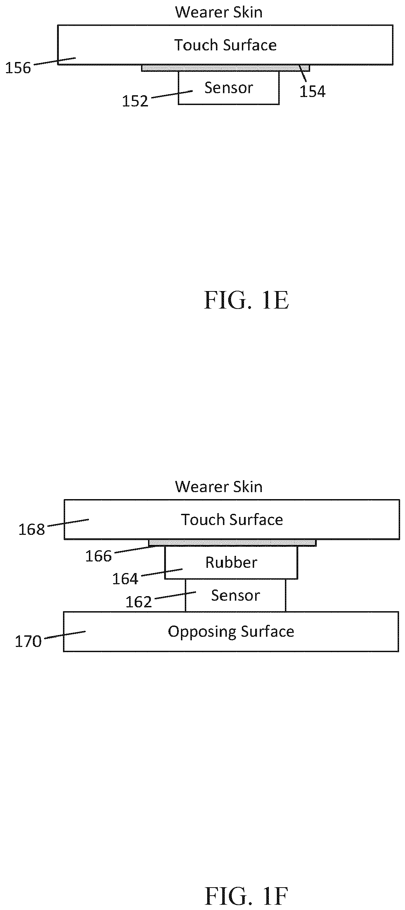

**FIG. 1E** is a schematic diagram that illustrates use of a strain gauge type sensor in accordance with the invention. As illustrated, a strain gauge sensor 152 is affixed via an adhesive layer 154 to a touch surface 156. In some implementations, touch surface 156 may correspond to a bottom cover of a wrist-worn device. For example, strain gauge sensor 152 may be affixed to a first side of touch surface 156 (e.g., in an interior portion of a capsule of the wrist-worn device), and a second (e.g., opposing) side of touch surface 156 may be configured to be in contact with a body portion of the wearer (e.g., a wrist of the wearer). It should be noted that, in some implementations, a strain gauge sensor may be affixed to a surface of which no side is configured to be in contact with the wearer. For example, strain gauge sensor 152 may be affixed to a side portion of a capsule, where the opposing end from where strain gauge sensor 152 is affixed corresponds to an end on which a band is configured to be coupled.

In some implementations, a pressure sensor may be a compression force sensor. A compression force sensor may be positioned between two surfaces and may be configured to detect a compression force between the two surfaces. For example, a compression force sensor may be positioned between a bottom cover of a wrist-worn device and another surface of the wrist-worn device (e.g., a PCB surface within an interior of a capsule of the wrist-worn device) such that the signal produced by the compression force sensor is directly proportional to a force of the bottom cover of the wrist-worn device on a body portion (e.g., a wrist) of the wearer.

**FIG. 1F** is a schematic diagram that illustrates use of a compression force sensor in accordance with some embodiments. As illustrated, a pressure sensor 162 may be affixed to a rubber element 164, which may in turn be affixed via adhesive 166 to a touch surface 168. Similar to what is described above in connection with FIG. 1E, touch surface 168 may correspond to a bottom cover of the wrist-worn device. For example, adhesive 166 may be on a first side of touch surface 168 (e.g., in an interior portion of a capsule of the wrist-worn device), and a second (e.g., opposing) side of touch surface 168 may be configured to be in contact with a body portion of the wearer (e.g., a wrist of the wearer). Rubber element 164 may serve to thermally and/or mechanically isolate pressure sensor 162 such that pressure sensor 162 is not affected by thermal variations on the portion of touch surface 168 that is physically in contact with the skin of the wearer. As illustrated in FIG. 1F, an opposing side of pressure sensor 162 is affixed to an opposing surface 170. Opposing surface 170 may be positioned within an interior of a capsule of the wrist-worn device. For example, opposing surface 170 may be a surface of a PCB within the capsule.

Wrist-worn devices, such as fitness trackers or smart watches, frequently measure physiological characteristics of a wearer. These physiological characteristics may include heart rate, oxygen saturation, blood pressure, or the like. These physiological characteristics may be determined using measurements from one or more sensors on-board the wrist-worn device, such as one or more light emitters, one or more light detectors, one or more accelerometers, one or more gyroscopes, etc. By way of example, PPG is an example of a technique that may be used to determine heart rate, oxygen saturation, blood pressure, etc. In PPG, light is emitted toward skin of the wearer, and the light is then reflected from the skin and/or from various internal body regions (e.g., blood vessels, blood cells, bone, etc.). The reflected light is then captured by one or more light detectors of the wrist-worn device, and characteristics of the reflected light, such as changes in absorption over different wavelengths of light, may be used to determine heart rate, oxygen saturation, blood pressure, etc.

In cases in which physiological characteristics are determined using sensors on board the wrist-worn device, a fit of the wrist-worn device is important. In particular, both too loose of a fit and too tight of a fit may result in inaccurate or sub-optimal measurements, which in turn yield inaccurate or sub-optimal physiological characteristic estimates. **FIG. 2** illustrates an example of optimal band tightness for physiological measurements that rely on detecting reflected light (e.g., using PPG) in accordance with the invention. As illustrated in panel 202, in an instance in which a back of the wrist-worn device (e.g., a surface of a bottom cover of the wrist-worn device) is not in contact with the body of the wearer, transmitted light may not reach blood vessels in the tissue of the wearer, and accordingly, reflected light may not reach a light detector within the wrist-worn device. This may lead to poor PPG data, and, in turn, poor physiological measurements determined based on the PPG data. Turning to panel 204, in an instance in which the back of the wrist-worn device is in contact with the body of the wearer, transmitted light from emitters of the wrist-worn device may pass through the skin of the wearer and reflect off of blood vessels carrying both venous blood and arterial blood. In this case, PPG-based physiological measurements may be possible, but, because measurements that include absorption of venous (e.g., non-oxygenated) blood may lead to less reliable results, band tightness that results in the wrist-worn device being merely in contact with the skin of the wearer may yield sub-optimal (e.g., inaccurate) results. Turning to panel 206, in an instance in which the band tightness is optimal, venous blood may be occluded, while allowing arterial blood to not be occluded. In this instance, by suppressing absorption measurements of venous blood, PPG-based measurements may be the most accurate. Turning to panel 208, in an instance in which the band of the wrist-worn device is too tight, both venous blood and arterial blood may be occluded. By also occluding arterial blood, PPG-based measurements may be less accurate than in an instance in which the band is less tight.

In other words, as illustrated in FIG. 2, there may be an optimal contact pressure of the wrist-worn device to the wrist of the wearer (and accordingly, an optimal band tightness) for physiological measurements. It should be noted that while FIG. 2 illustrates an example of optimal contact pressure in an instance in which physiological measurements are made using characteristics of measured reflected light, an optimal band tightness may exist for physiological measurements that are made using other techniques, such as based on accelerometers, or the like. Moreover, an optimal contact pressure may vary based on multiple factors, such as the type of sensors being used, the type of physiological measurement being performed, an ambient temperature, whether the wearer is in motion (e.g., walking, running, etc.), whether the wearer is sleeping, or the like.

In some implementations, a current contact pressure of a wrist-worn device to a wrist of a wearer may be determined using one or more pressure sensors of the wrist-worn device. The current contact pressure may be compared to a target contact pressure. The target contact pressure may be different for different types of physiological measurements, for different people, at different ambient temperatures or humidity levels, during different activities (e.g., sleeping, exercising, working at a computer, etc.), and/or vary based on any other criteria. In some implementations, target contact pressure may be determined for an individual based on a calibration process performed while the individual is wearing the wrist-worn device. Additionally or alternatively, the target contact pressure may be determined based on data collected from multiple users. In some examples, based on a difference between a target contact pressure and a current contact pressure, instructions may be provided to the wearer to alter a tightness of the wrist-worn device to achieve the target contact pressure. For example, instructions may be provided to tighten the band of the wrist-worn device (e.g., by a suggested number of notches), loosen the band of the wrist-worn device (e.g., by a suggested number of notches), or the like.

**FIG. 3** is a flowchart of an example process 300 to provide instructions to alter a tightness of a wrist-worn device in accordance with the invention. In some implementations, blocks of process 300 may be performed by one or more processors. The one or more processors may be on-board the wrist-worn device. Additionally or alternatively, the one or more processors may be on a user device (e.g., a mobile phone, a tablet computer, or the like) that is paired with the wrist-worn device, on a server device, or any other suitable device. In some implementations, blocks of process 300 may be executed in an order other than what is shown in FIG. 3. In some implementations, two or more blocks of process 300 may be performed substantially in parallel. In some examples, one or more blocks of process 300 may be omitted.

Process 300 can begin at 302 by obtaining sensor data from one or more pressure sensors disposed in and/or on a wrist-worn device. As shown in and described above in connection with FIGS. 1A-1D, the one or more pressure sensors may be disposed proximate to a back cover of the wrist-worn device, proximate to a side of a capsule of the wrist-worn device, proximate to a band of the wrist-worn device, and/or in any other suitable location(s). As described above in connection with FIGS. 1E and 1F, the one or more pressure sensors may be strain gauge type sensors, compression force sensors, and/or a combination of strain gauge sensors and compression force sensors. The sensor data may be obtained by a processor, which may be on-board the wrist-worn device or remote from the wrist-worn device. For example, the sensor data may be transmitted from the wrist-worn device to a user device paired with the wrist-worn device for subsequent analysis and processing. As another example, the sensor data may be transmitted from the wrist-worn device to a server device for subsequent analysis and processing.

At 304, process 300 can determine a contact pressure of the wrist-worn device to a wrist of the wearer based on the sensor data. For example, in instances in which the one or more pressure sensors include a strain gauge type sensor, process 300 can determine the contact pressure based on an amount of deflection of a surface the strain gauge type sensor is affixed to. As another example, in instances in which the one or more pressure sensors include a compression force type sensor, process 300 can determine the contact pressure based on an amount of compression experienced by the compression force sensor. In some implementations, process 300 may determine contact pressure by providing the sensor data to a look up table or algorithm (e.g., one or more mathematical functions) that translate the sensor data to a contact pressure measurement.

In instances in which the sensor data includes sensor data from two or more pressure sensors, process 300 may combine data from the two or more pressure sensors. For example, in some implementations, process 300 may determine an aggregate contact pressure by averaging or combining the data from the two or more pressure sensors. In some implementations, process 300 may aggregate sensor data from two or more pressure sensors responsive to the two or more pressure sensors being of the same sensor type (e.g., strain gauge sensors or compression force sensors). As another example, in some implementations, process 300 may utilize the sensor data from the two or more pressure sensors to determine whether the capsule of the wrist-worn device is tilted in a particular direction. As a more particular example, in an instance in which two pressure sensors are disposed at opposite sides of a back cover of the wrist-worn device, and in which one of the two pressure sensors outputs sensor data indicating greater contact pressure than the other pressure sensor, process 300 may determine that the capsule of the wrist-worn device is tilted toward the pressure sensor providing increased contact pressure measurements.

At 306, process 300 can identify a target contact pressure of the wrist-worn device. In some implementations, the target contact pressure may be identified based on a physiological measurement that is to be performed, types of sensors to be used for physiological measurements, a current activity state of the wearer, a current ambient temperature or humidity, and/or any suitable combination of factors. Examples of types of physiological measurements that may be considered when identifying a target contact pressure include heart rate, oxygen saturation, blood pressure, EMG sensing, and/or body temperature. Examples of types of sensors to be used for physiological measurements include light emitters, light detectors, accelerometers, gyroscopes, surface EMG electrodes, or the like. In some embodiments, a wavelength of light that is to be emitted and/or captured in a reflection may be considered when determining the target contact pressure. Examples of activity states may include walking, running, sitting, typing, sleeping, or the like.

Process 300 may determine the target contact pressure in a manner that is specific to the wearer of the wrist-worn device. For example, a calibration technique may have previously been performed for the wearer of the wrist-worn device to determine target contact pressures for different physiological measurements, under different activity states, at different ambient temperatures or humidities, or the like. In some such examples, the calibration technique may involve performing one or more physiological measurements (e.g., measuring heart rate, oxygen saturation, or the like) with varying band tightness. For example, the calibration technique may involve instructing the user to adjust the band to their preferred tightness, performing a physiological measurement, instructing the user to adjust the band to tighter and/or looser states, and re-performing the physiological measurement. Continuing with this example, the optimal band tightness for the physiological measurement performed may be identified as the band tightness corresponding to the contact pressure under which the physiological measurement satisfies various criteria (e.g., variance in a sequence of physiological measurements being below a predetermined threshold, or the like). The calibration technique may be repeated for the wearer under different ambient temperature conditions.

In some implementations, process 300 may determine the target contact pressure based on data collected from multiple users other than the wearer of the wrist-worn device. For example, physiological measurements may be collected from wrist-worn devices of the multiple users and stored in connection with other data indicating, e.g., the contact pressure of the wrist-worn device at the time the physiological measurement was made, the ambient temperature and/or humidity at a time the physiological measurement was made, the activity the wearer was engaged in at the time the physiological measurement was made, or the like. Continuing with this example, the physiological measurements may be scored or evaluated for quality based on various criteria such as a likelihood the physiological measurement is accurate (e.g., based on whether the physiological measurement is a plausible value), variance of a sequence of physiological measurements collected in a relatively short duration of time (e.g., within five seconds, within ten seconds, within thirty seconds, within a minute, or the like), and/or any other criteria. Continuing still further with this example, the target contact pressure may be identified as one that is associated with higher-scored physiological measurements from the multiple users. In other words, the target contact pressure may be identified as one that is more likely to yield higher quality physiological measurements. One or more machine learning models may be trained using the data from the multiple users, and the target contact pressure may be obtained by providing input indicative of one or more physiological measurements to be performed, types of sensors to be used to perform the one or more physiological measurements, a current activity state of the wearer, and/or ambient weather conditions, where the target contact pressure is an output of the machine learning model.

At 308, process 300 may provide instructions to the wearer to adjust the wrist-worn device to achieve the identified target contact pressure. For example, in an instance in which the contact pressure detected at block 304 is greater than the target contact pressure identified at block 306, process 300 may present instructions that instruct the user to loosen a band of the wrist-worn device to decrease the current contact pressure. Conversely, in an instance in which the contact pressure detected at block 304 is less than the target contact pressure identified at block 306, process 300 may present instructions that instruct the user to tighten a band of the wrist-worn device to increase the contact pressure. In some examples, the instructions may include an indication of the magnitude of the adjustment. For example, the instructions may indicate a number of notches the band is to be loosened or tightened. By way of example, the instructions may state "increase the band tightness by one notch," or the like. The magnitude of the adjustment may be determined based on a degree to which the current contact pressure differs from the identified target contact pressure, with larger adjustments being indicated for larger differences between the current contact pressure and the target contact pressure, and vice versa.

The instructions may be presented on a display of the wrist-worn device. Additionally or alternatively, the instructions may be presented on a display of a user device paired with the wrist-worn device, such as a display of a paired mobile phone or tablet computer. In some implementations, the instructions may be presented in connection with any suitable type of alert, such as haptic feedback, an audible tone, or the like.

In some implementations, additionally or alternatively to providing instructions to adjust a tightness of a band of a wrist-worn device, one or more changes may be made while performing physiological measurements based on a current contact pressure of the wrist-worn device to the wrist of the wearer. The one or more changes made while performing physiological measurements may allow for closed-loop control of physiological measurements, thereby causing the physiological measurements to be more accurate. In some implementations, the one or more changes may include changes to hardware. For example, the one or more changes may include increased current of an LED used to emit light toward tissue of the wearer, such as that used in connection with PPG-based measurements. In some implementations, the one or more changes may include changes to a manner in which physiological measurements are determined. For example, in an instance in which a physiological measurement (e.g., heart rate, oxygen saturation, blood pressure, EMG, or the like) is determined based on collected sensor data (e.g., data collected from one or more light detectors, data collected from one or more EMG electrodes, or the like), the one or more changes may include integrating the collected sensor data over a longer than usual duration of time to, for example, increase signal to noise ratio. In some implementations, the one or more changes may include augmenting sensor data used to determine the physiological measurements with other data, such as body temperature data, motion sensor data, or the like, which may improve an accuracy of the physiological measurements. In some implementations, responsive to determining that the current contact pressure deviates from a target contact pressure by more than a threshold amount, physiological measurements may be reported in an altered manner, for example, with an indication that the physiological measurements may not be accurate due to inappropriate band tightness of the wrist-worn device. Alternatively, in some implementations, physiological measurements may be discarded (e.g., not stored and/or not provided to the wearer) responsive to the current contact pressure deviating from the target contact pressure by more than the threshold amount.

**FIG. 4** is a flowchart of an example process 400 for closed-loop control of physiological measurements based on contact pressure of a wrist-worn device to a wrist of a wearer in accordance with the invention. In some implementations, process 400 may be performed by one or more processors. The one or more processors may be on-board the wrist-worn device. Additionally or alternatively, at least one of the one or more processors may be on a user device (e.g., a mobile phone, a tablet computer, or the like) paired with the wrist-worn device, a server, etc. In some embodiments, blocks of process 400 may be performed in an order other than what is shown in FIG. 4. In some examples, two or more blocks of process 400 may be performed substantially in parallel. In some examples one or more blocks of process 400 may be omitted.

Process 400 can begin at 402 by obtaining sensor data from one or more pressure sensors disposed in and/or on a wrist-worn device. Similar to what is described above in connection with block 302 of FIG. 3, the one or more pressure sensors may be disposed at any suitable locations of the wrist-worn device, and the one or more pressure sensors may be strain gauge type sensors, compression force type sensors, or any combination thereof.

At 404, process 400 can determine a contact pressure of the wrist-worn device to a wrist of the wearer based on the sensor data. Similar to what is described above in connection with block 304 of FIG. 3, the contact pressure may be determined using the sensor data by, for example, providing the sensor data to an algorithm or look-up table that translates the sensor data to a corresponding contact pressure. Additionally or alternatively, as described above in connection with block 304 of FIG. 3, data from multiple pressure sensors may be combined to determine an aggregate contact pressure and/or to determine a tilt of the capsule of the wrist-worn device relative to the wrist of the wearer.

At 406, process 400 can identify a target contact pressure of the wrist-worn device for a type of physiological measurement to be made using sensors of the wrist-worn device. Examples of types of physiological measurements include heart rate, oxygen saturation, blood pressure, respiration rate, muscle activity measurements (e.g., EMG measurements), and/or body temperature. In some implementations, process 400 may identify the target contact pressure for the type of physiological measurement by accessing a look-up table that specifies a target contact pressure for the particular type of physiological measurement. Additionally or alternatively, process 400 may identify the target contact pressure by providing the type of physiological measurement in connection with any other relevant information (e.g., current ambient weather conditions, current activity state of the wearer, etc.) to a machine learning model that generates the target contact pressure as an output. It should be noted that, in some implementations, a look-up table, a trained machine learning model, and/or any other source of the target contact pressure may be specific to the wearer of the wrist-worn device. In such instances, the look-up table, the trained machine learning model, or the like, may be generated based on a calibration routine performed with the wearer of the wrist-worn device, as described above in connection with block 306 of FIG. 3.

At 408, process 400 can determine at least one change to be made when performing the physiological measurement based on a difference between the contact pressure determined at block 404 and the target contact pressure identified at block 406. In some implementations, the at least one change made depend on a direction of the difference (e.g., whether the contact pressure is greater than or less than the target contact pressure), a degree of the difference, and/or the type of physiological measurement. For example, in instances in which the type of physiological measurement is a PPG-based measurement (e.g., determining heart rate based on a PPG signal, determining blood pressure based on a PPG signal, determining oxygen saturation based on a PPG signal, or the like), the at least one change may include changes to a light emitter used to emit light toward the tissue of the wearer. As a more particular example, the at least one change may include a change in LED current (e.g., an increase in LED current) to cause the light emitted toward the tissue of the wearer to be brighter/more intense, which may ameliorate negative effects of a contact pressure that differs from the target contact pressure. In some implementations, the degree of change in LED current may correspond to a degree to which the contact pressure differs from the target contact pressure. For example, LED current may be changed by a greater amount in instances in which the contact pressure differs from the target contact pressure by a greater amount, and vice versa. As a more particular example, in instances in which the contact pressure is less than the target contact pressure, the LED current may be increased to overcome negative effects from a back cover of the wrist-worn device that is poorly in contact with the wrist of the wearer (e.g., as shown in and described above in connection with panel 204 of FIG. 2). As another more particular example, in instances in which the contact pressure is greater than the target contact pressure, the LED current may be decreased to overcome negative effects from a too-tight wrist-worn device (e.g., as shown in and described above in connection with panel 208 of FIG. 2).

As another example, in some implementations, the at least one change can include integrating sensor data used to perform the physiological measurement over an increased time duration relative to the typical integration time duration. By integrating sensor data over a longer time period, noise in the sensor data may be averaged out, thereby causing increased signal to noise ratio in the sensor data used to determine the physiological measurement. The time duration may be increased in cases where the contact pressure is greater than the target contact pressure and in instances in which the contact pressure is less than the target contact pressure. The integration time may be changed based on the type of physiological measurement to be performed. For example, the integration time may be changed by a first amount in instances in which the physiological measurement corresponds to a first type of physiological measurement (e.g., heart rate), and by a second amount in instances in which the physiological measurement corresponds to a second type of physiological measurement (e.g., oxygen saturation).

As yet another example, the at least one change can include considering other data when determining the physiological measurement. For example, the other data may include motion data (e.g., obtained from one or more accelerometers and/or gyroscopes of the wrist-worn device) and/or body temperature data (e.g., obtained from one or more temperature sensors of the wrist-worn device). By way of example, in an instance in which it is determined that the contact pressure differs from the target contact pressure by more than a threshold amount, and in which the physiological measurement to be made includes a heart rate of the wearer, motion data may be considered when determining the heart rate. This may compensate for effects from, e.g., a too-loose band and the wrist-worn device slipping on a wrist of the wearer during exercise. As another example, in an instance in which it is determined that the contact pressure differs from the target contact pressure by more than a threshold amount, and in which the physiological measurement to be made includes EMG measurements (e.g., of finger, wrist, hand, and/or arm muscles), motion data may be considered when determining the EMG activity. This may compensate for effects from, e.g., a too loose or too tight band precluding acquisition of robust EMG signals during hand, finger, or wrist motion.

At 410, process 400 can perform the physiological measurements while implementing the at least one change. For example, in instances in which the physiological measurements involve PPG-based measurements, process 400 can cause light to be emitted toward a tissue of the wearer, obtain measurements indicating light reflected from the tissue of the wearer, and determine the physiological measurement based on the obtained measurements while implementing the at least one change (e.g., a change in the LED current of the light emitter, integrating outputs of the light detector over a longer time period, considering motion sensor data, considering temperature sensor data, etc.). As another example, in instances in which the physiological measurements involve EMG measurements, process 400 can receive outputs from one or more EMG electrodes and determine EMG activity of one or more muscles while implementing the at least one change (e.g., integrating outputs of the EMG electrodes over a longer time period, considering motion sensor data, considering temperature sensor data, etc.). The other data (e.g., motion data, temperature data, or the like) may be considered by providing the other data to a machine learning model that takes the sensor data (e.g., light detector data, EMG electrode data, or the like) as well as the other data to generate the physiological measurement.

The at least one change made when performing the physiological measurement may be maintained during subsequent physiological measurements. For example, the at least one change may be maintained until the contact pressure changes (e.g., to be closer to the target contact pressure). As another example, the at least one change may be maintained until at least one other condition changes, such as a change in the activity state of the wearer, a change in the ambient weather conditions, or the like. Continuing with this example, responsive to detecting the at least one other condition change, a determination of whether to continue the at least one change made when performing the physiological measurement may be made. By way of example, in an instance in which LED current was increased during PPG-based measurements, the increased LED current may be used during a current activity state of the user (e.g., during exercise), and use of the increased LED current during PPG-based measurements may be re-evaluated responsive to detecting a change in the current activity state (e.g., during rest).

In some implementations, contact pressure information may be utilized to determine at least one physiological measurement. For example, the contact pressure information may indicate pulsatile pressure changes imparted to the back cover of the wrist-worn device by pulsatile changes in a user's artery under the wrist-worn device (e.g., the radial artery or the ulnar artery). In particular, for every heartbeat, blood ejected from the aorta of the heart may cause an upward then subsequent downward pulsatile motion of distal arteries (e.g., the radial artery and/or the ulnar artery in the wrist), where the pulsatile motion of the artery may be transferred to the back cover of the wrist-worn device and detected and/or characterized by force sensors proximate the back cover. The contact pressure information may be considered ballistocardiogram information.

The contact pressure information may be used to determine heart rate. For example, the contact pressure information may correspond to ballistocardiograph data. Continuing with this example, a heart rate may be determined from the ballistocardiograph data based on, e.g., a periodicity in the ballistocardiograph data. By way of example, a heart rate may be determined to be the inverse of a period of a ballistocardiogram signal. Periodicity information may be determined using spectral analysis techniques or the like. As another example, the contact pressure information may be used to determine other physiological measurements, such as blood pressure or oxygen saturation, in conjunction with PPG data. As a more particular example, the contact pressure information may be used to calibrate the physiological measurement determined using PPG data. As another more particular example, because the contact pressure information is in pressure or force units, the contact pressure information may be used to scale a blood pressure measurement from a relative pressure determined using the PPG data to an absolute blood pressure.

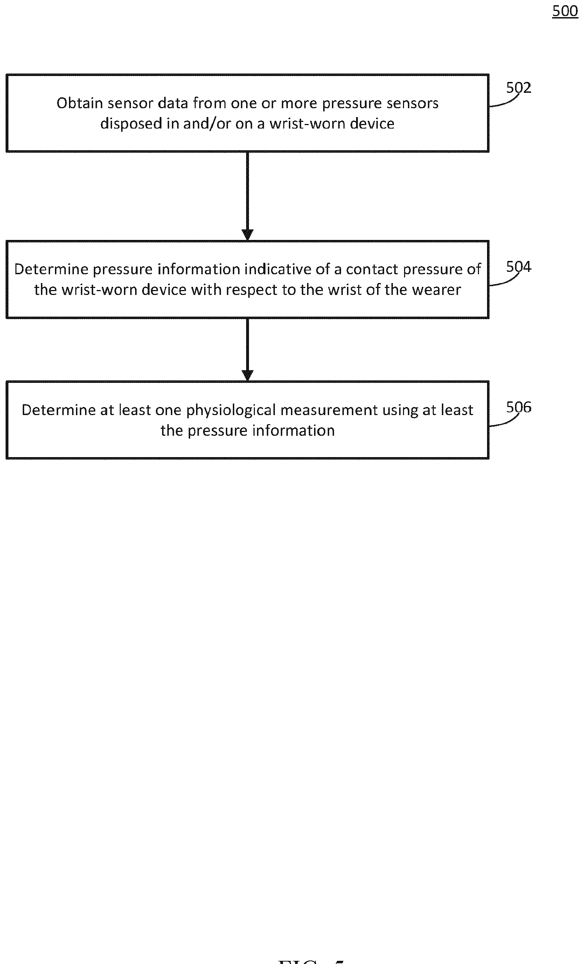

**FIG. 5** is a flowchart of an example process 500 for determining physiological measurements using contact pressure information. In some implementations, blocks of process 500 may be implemented using one or more processors or controllers of a wrist-worn device. In some examples, two or more blocks of process 500 may be executed substantially in parallel. In some examples, one or more blocks of process 500 may be omitted. In some examples, blocks of process 500 may be executed in an order other than what is shown in FIG. 5.

Process 500 can begin at 502 by obtaining sensor data from one or more pressure sensors disposed in and/or on a wrist-worn device. Similar to what is described above in connection with block 302 of FIG. 3, the one or more pressure sensors may be disposed at any suitable locations of the wrist-worn device, and the one or more pressure sensors may be strain gauge type sensors, compression force type sensors, or any combination thereof.

At 504, process 500 can determine pressure information indicative of a contact pressure of the wrist-worn device with respect to the wrist of the wearer. Similar to what is described above in connection with block 304 of FIG. 3, the contact pressure may be determined using the sensor data by, for example, providing the sensor data to an algorithm or look-up table that translates the sensor data to a corresponding contact pressure. Additionally or alternatively, as described above in connection with block 304 of FIG. 3, data from multiple pressure sensors may be combined to determine an aggregate contact pressure and/or to determine a tilt of the capsule of the wrist-worn device relative to the wrist of the wearer.

At 506, process 500 can determine at least one physiological measurement using at least the pressure information. Examples of physiological measurements include a heart rate, a blood pressure, an oxygen saturation, a heart rate variability, or the like. In one example, the pressure information may represent a ballistocardiogram. A heart rate may be determined using periodicity information of the ballistocardiogram, e.g., by determining the heart rate as the inverse of the period of the heart rate signal represented in the ballistocardiogram.

In some implementations, information derived from the pressure information may be used to determine the physiological measurement. For example, in an instance in which the pressure information represents a ballistocardiogram, cardiac cycle reference points or biomarkers may be identified in the ballistocardiogram. Timing information associated with these biomarkers may then be used to determine or calibrate various physiological measurements, such as a blood pressure, oxygen saturation, etc. Examples of biomarkers that may be identified in the ballistocardiogram include the diastolic point, the dicrotic notch, or the like. In some implementations, timing information associated with a biomarker identified in the ballistocardiogram (e.g., obtained from the pressure information) may be used in conjunction with a PPG signal to determine a physiological measurement. For example, the PPG signal may be annotated with the identified biomarkers, and the physiological measurement (e.g., blood pressure, oxygen saturation, etc.) may be identified based on the annotated PPG signal.

In instances in which the at least one physiological measurement includes blood pressure, the pressure information may be used to calibrate a relative blood pressure determined using a PPG signal. For example, a PPG signal may be used to determine a relative blood pressure, and the pressure information may be used to modify the relative blood pressure measurement obtained using PPG to units of pressure (e.g., mmHG). In other words, because the pressure information is in units of force or pressure, the pressure information may be used to calibrate or scale a relative blood pressure obtained using PPG to an absolute blood pressure in units of mmHG.

It should be understood that, in an instance in which the pressure information is utilized in conjunction with a PPG signal to determine the at least one physiological measurement, the PPG signal may be obtained from a device other than the wrist-worn device. For example, the other device may be a user device that is paired with or otherwise associated with (e.g., via a BLUETOOTH connection or other wireless communication channel) with the wrist-worn device. Examples of other devices include: smart glasses, a virtual reality (VR) or augmented reality (AR) headset, a mobile phone, a wearable ring, etc. By way of example, in an instance in which the other device is a pair of smart glasses or a VR/AR headset, the PPG signal may be obtained by utilizing a light emitter and/or a light detector disposed in a frame of the glasses/headset such that the light is emitted toward a portion of the wearer's forehead or the side of their head, toward the user's ear, etc.

The methods, systems, apparatuses, and media described herein may be used in conjunction with various technologies, such as an artificial reality system. An artificial reality system, such as a head-mounted display (HMD) or heads-up display (HUD) system, generally includes a display configured to present artificial images that depict objects in a virtual environment. The display may present virtual objects or combine images of real objects with virtual objects, as in virtual reality (VR), augmented reality (AR), or mixed reality (MR) applications. For example, in an AR system, a user may view both displayed images of virtual objects (e.g., computer-generated images (CGIs)) and the surrounding environment by, for example, seeing through transparent display glasses or lenses (often referred to as optical see-through) or viewing displayed images of the surrounding environment captured by a camera (often referred to as video see-through). In some AR systems, the artificial images may be presented to users using an LED-based display subsystem.

The methods, systems, apparatuses, and media described herein may be implemented in connection with a wearable computer, such as a smart watch, a fitness tracker, a HMD, or the like. For example, such a wearable computer may include one or more light emitters and/or one or more light sensors incorporated into a portion of an enclosure of the wearable computer such that light can be emitted toward a tissue of a wearer of the wearable computer that is proximate to or touching the portion of the enclosure of the wearable computer. Example locations of such a portion of an enclosure of a wearable computer may include a portion configured to be proximate to an ear of the wearer (e.g., proximate to a superior tragus, proximate to a superior auricular, proximate to a posterior auricular, proximate to an inferior auricular, or the like), proximate to a forehead of the wearer, proximate to a wrist of the wearer, proximate to a finger tip of the wearer, proximate to a base of a finger of a wearer, proximate to a toe tip of a wearer, or the like.

In the following description, for the purposes of explanation, specific details are set forth in order to provide a thorough understanding of examples of the disclosure. However, it will be apparent that various examples may be practiced without these specific details. For example, devices, systems, structures, assemblies, methods, and other components may be shown as components in block diagram form in order not to obscure the examples in unnecessary detail. In other instances, well-known devices, processes, systems, structures, and techniques may be shown without necessary detail in order to avoid obscuring the examples. The figures and description are not intended to be restrictive. The terms and expressions that have been employed in this disclosure are used as terms of description and not of limitation, and there is no intention in the use of such terms and expressions of excluding any equivalents of the features shown and described or portions thereof. The word "example" is used herein to mean "serving as an example, instance, or illustration." Any embodiment or design described herein as "example" is not necessarily to be construed as preferred or advantageous over other embodiments or designs.

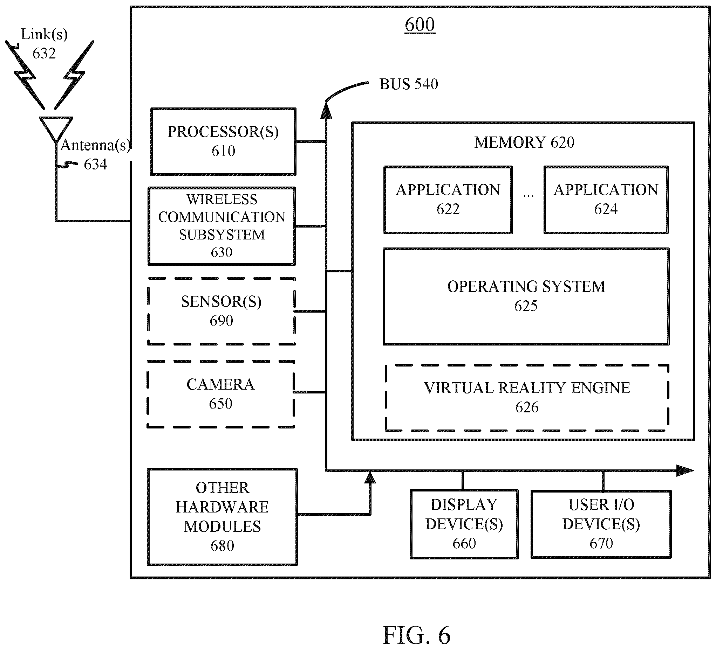

**FIG. 6** is a simplified block diagram of an example of a computing system 600 for implementing some of the examples described herein. For example, computing system may be used to implement a user device (e.g., a mobile phone, a tablet computer, a wrist-worn device, etc.) that implements the blocks of process 300 and/or process 400 shown in and described above in connection with FIGS. 3 and 4. In the illustrated example, computing system 600 may include one or more processor(s) 610 and a memory 620. Processor(s) 610 may be configured to execute instructions for performing operations at a number of components, and can be, for example, a general-purpose processor or microprocessor suitable for implementation within a portable electronic device. Processor(s) 610 may be communicatively coupled with a plurality of components within computing system 600. To realize this communicative coupling, processor(s) 610 may communicate with the other illustrated components across a bus 640. Bus 640 may be any subsystem adapted to transfer data within computing system 600. Bus 640 may include a plurality of computer buses and additional circuitry to transfer data.

Memory 620 may be coupled to processor(s) 610. Memory 620 may offer both short-term and long-term storage and may be divided into several units. Memory 620 may be volatile, such as static random access memory (SRAM) and/or dynamic random access memory (DRAM) and/or non-volatile, such as read-only memory (ROM), flash memory, and the like. Furthermore, memory 620 may include removable storage devices, such as secure digital (SD) cards. Memory 620 may provide storage of computer-readable instructions, data structures, program modules, and other data for computing system 600. Memory 620 may be distributed into different hardware modules. A set of instructions and/or code might be stored on memory 620. The instructions might take the form of executable code that may be executable by computing system 600, and/or might take the form of source and/or installable code, which, upon compilation and/or installation on computing system 600 (e.g., using any of a variety of generally available compilers, installation programs, compression/decompression utilities, etc.), may take the form of executable code.

Memory 620 may store a plurality of application modules 622 through 624, which may include any number of applications. Examples of applications may include gaming applications, conferencing applications, video playback applications, or other suitable applications. The applications may include a depth sensing function or eye tracking function. Application modules 622-624 may include particular instructions to be executed by processor(s) 610. Certain applications or parts of application modules 622-624 may be executable by other hardware modules 680. In certain embodiments, memory 620 may additionally include secure memory, which may include additional security controls to prevent copying or other unauthorized access to secure information.

Memory 620 may include an operating system 625 loaded therein. Operating system 625 may be operable to initiate the execution of the instructions provided by application modules 622-624 and/or manage other hardware modules 680 as well as interfaces with a wireless communication subsystem 630 which may include one or more wireless transceivers. Operating system 625 may be adapted to perform other operations across the components of computing system 600 including threading, resource management, data storage control and other similar functionality.

Wireless communication subsystem 630 may include, for example, an infrared communication device, a wireless communication device and/or chipset (such as a Bluetooth^{®} device, an IEEE 802.11 device, a Wi-Fi device, a WiMax device, cellular communication facilities, etc.), and/or similar communication interfaces. Computing system 600 may include one or more antennas 634 for wireless communication as part of wireless communication subsystem 630 or as a separate component coupled to any portion of the system. Depending on desired functionality, wireless communication subsystem 630 may include separate transceivers to communicate with base transceiver stations and other wireless devices and access points, which may include communicating with different data networks and/or network types, such as wireless wide-area networks (WWANs), wireless local area networks (WLANs), or wireless personal area networks (WPANs). A WWAN may be, for example, a WiMax (IEEE 802.15) network. A WLAN may be, for example, an IEEE 802.11x network. A WPAN may be, for example, a Bluetooth network, an IEEE 802.5x, or some other types of network. The techniques described herein may also be used for any combination of WWAN, WLAN, and/or WPAN. Wireless communications subsystem 630 may permit data to be exchanged with a network, other computer systems, and/or any other devices described herein. Wireless communication subsystem 630 may include a means for transmitting or receiving data, such as identifiers of HMD devices, position data, a geographic map, a heat map, photos, or videos, using antenna(s) 634 and wireless link(s) 632. Wireless communication subsystem 630, processor(s) 610, and memory 620 may together comprise at least a part of one or more of a means for performing some functions disclosed herein.

Examples of computing system 600 may also include one or more sensors 690. Sensor(s) 690 may include, for example, an image sensor, an accelerometer, a pressure sensor, a temperature sensor, a proximity sensor, a magnetometer, a gyroscope, an inertial sensor (e.g., a module that combines an accelerometer and a gyroscope), an ambient light sensor, or any other similar module operable to provide sensory output and/or receive sensory input, such as a depth sensor or a position sensor. For example, in some implementations, sensor(s) 690 may include one or more inertial measurement units (IMUs) and/or one or more position sensors. An IMU may generate calibration data indicating an estimated position of a device, based on measurement signals received from one or more of the position sensors. A position sensor may generate one or more measurement signals in response to motion of a device. Examples of the position sensors may include, but are not limited to, one or more accelerometers, one or more gyroscopes, one or more magnetometers, another suitable type of sensor that detects motion, a type of sensor used for error correction of the IMU, or some combination thereof. The position sensors may be located external to the IMU, internal to the IMU, or some combination thereof. At least some sensors may use a structured light pattern for sensing.

Computing system 600 may include a display module 660. Display module 660 may be a near-eye display, and may graphically present information, such as images, videos, and various instructions, from computing system 600 to a user. Such information may be derived from one or more application modules 622-624, virtual reality engine 626, one or more other hardware modules 680, a combination thereof, or any other suitable means for resolving graphical content for the user (e.g., by operating system 625). Display module 660 may use liquid crystal display (LCD) technology, light-emitting diode (LED) technology (including, for example, OLED, ILED, mLED, AMOLED, TOLED, etc.), light emitting polymer display (LPD) technology, or some other display technology.

Computing system 600 may include a user input/output module 670. User input/output module 670 may allow a user to send action requests to computing system 600. An action request may be a request to perform a particular action. For example, an action request may be to start or end an application or to perform a particular action within the application. User input/output module 670 may include one or more input devices. Example input devices may include a touchscreen, a touch pad, microphone(s), button(s), dial(s), switch(es), a keyboard, a mouse, a game controller, or any other suitable device for receiving action requests and communicating the received action requests to computing system 600. User input/output module 670 may provide haptic feedback to the user in accordance with instructions received from computing system 600. For example, the haptic feedback may be provided when an action request is received or has been performed.

Computing system 600 may include a camera 650 that may be used to take photos or videos. Camera 650 may be configured to take photos or videos of the user. Camera 650 may also be used to take photos or videos of the environment, for example, for VR, AR, or MR applications. Camera 650 may include, for example, a complementary metal-oxide-semiconductor (CMOS) image sensor with a few millions or tens of millions of pixels. In some implementations, camera 650 may include two or more cameras that may be used to capture 3-D images.

Computing system 600 may include a plurality of other hardware modules 680. Each of other hardware modules 680 may be a physical module within computing system 600. While each of other hardware modules 680 may be permanently configured as a structure, some of other hardware modules 680 may be temporarily configured to perform specific functions or temporarily activated. Examples of other hardware modules 680 may include, for example, an audio output and/or input module (*e.g*., a microphone or speaker), a near field communication (NFC) module, a rechargeable battery, a battery management system, a wired/wireless battery charging system, etc. In some embodiments, one or more functions of other hardware modules 680 may be implemented in software.

Memory 620 of computing system 600 may also store a virtual reality engine 626. Virtual reality engine 626 may execute applications within computing system 600 and receive position information, acceleration information, velocity information, predicted future positions, or some combination thereof from the various sensors. The information received by virtual reality engine 626 may be used for producing a signal (*e.g*., display instructions) to display module 660. For example, if the received information indicates that the user has looked to the left, virtual reality engine 626 may generate content that mirrors the user's movement in a virtual environment. Additionally, virtual reality engine 626 may perform an action within an application in response to an action request received from user input/output module 670 and provide feedback to the user. The provided feedback may be visual, audible, or haptic feedback. In some implementations, processor(s) 610 may include one or more GPUs that may execute virtual reality engine 626.

In various implementations, the above-described hardware and modules may be implemented on a single device or on multiple devices that can communicate with one another using wired or wireless connections. For example, in some implementations, some components or modules, such as GPUs, virtual reality engine 626, and applications (*e.g*., tracking application), may be implemented on two or more paired or connected devices.

In alternative configurations, different and/or additional components may be included in computing system 600. Similarly, functionality of one or more of the components can be distributed among the components in a manner different from the manner described above. For example, computing system 600 may be modified to include other system environments, such as an AR system environment and/or an MR environment.

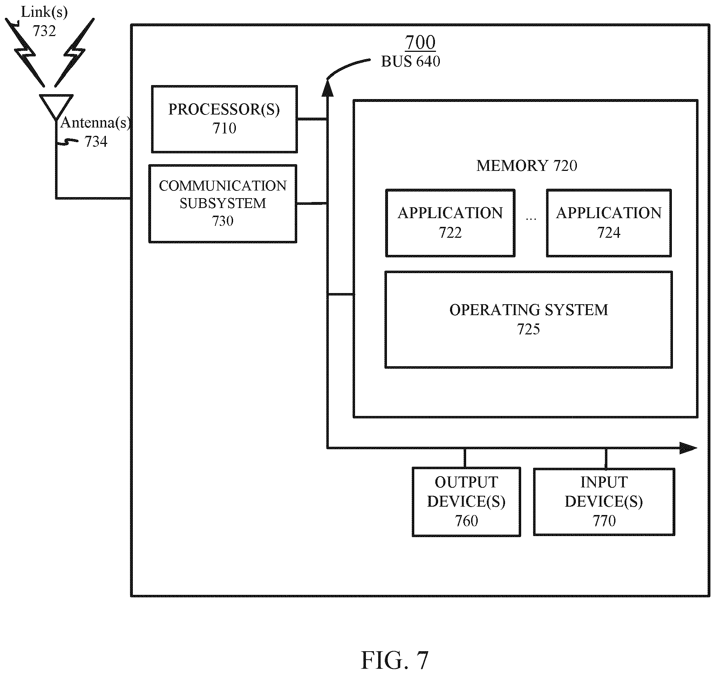

FIG. 7 is a simplified block diagram of an example of a computing system 700 that may be implemented in connection with a server. For example, computing system 700 may be used to implement a server that generates a trained machine learning model that identifies target contact pressures, stores data related to contact pressures of wrist-worn devices of multiple users, or the like.

In the illustrated example, computing system 700 may include one or more processor(s) 710 and a memory 720. Processor(s) 710 may be configured to execute instructions for performing operations at a number of components, and can be, for example, a general-purpose processor or microprocessor suitable for implementation within a portable electronic device. Processor(s) 710 may be communicatively coupled with a plurality of components within computing system 700. To realize this communicative coupling, processor(s) 710 may communicate with the other illustrated components across a bus 740. Bus 740 may be any subsystem adapted to transfer data within computing system 700. Bus 740 may include a plurality of computer buses and additional circuitry to transfer data. Processor(s) 710 may be configured to perform one or more blocks of process 400, as shown in and described above in connection with FIG. 4, respectively.

Memory 720 may be coupled to processor(s) 710. Memory 720 may offer both short-term and long-term storage and may be divided into several units. Memory 720 may be volatile, such as static random access memory (SRAM) and/or dynamic random access memory (DRAM) and/or non-volatile, such as read-only memory (ROM), flash memory, and the like. Furthermore, memory 720 may include removable storage devices, such as secure digital (SD) cards. Memory 720 may provide storage of computer-readable instructions, data structures, program modules, and other data for computing system 700. In some examples, memory 720 may be distributed into different hardware modules. A set of instructions and/or code might be stored on memory 720. The instructions might take the form of executable code that may be executable by computing system 700, and/or might take the form of source and/or installable code, which, upon compilation and/or installation on computing system 700 (e.g., using any of a variety of generally available compilers, installation programs, compression/decompression utilities, etc.), may take the form of executable code.

Memory 720 may store a plurality of application modules 722 through 724, which may include any number of applications. Examples of applications may include gaming applications, conferencing applications, video playback applications, or other suitable applications. Application modules 722-724 may include particular instructions to be executed by processor(s) 710. Certain applications or parts of application modules 722-724 may be executable by other hardware modules. Memory 720 may additionally include secure memory, which may include additional security controls to prevent copying or other unauthorized access to secure information.

Memory 720 may include an operating system 725 loaded therein. Operating system 725 may be operable to initiate the execution of the instructions provided by application modules 722-724 and/or manage other hardware modules as well as interfaces with a wireless communication subsystem 730 which may include one or more wireless transceivers. Operating system 725 may be adapted to perform other operations across the components of computing system 700 including threading, resource management, data storage control and other similar functionality.

Communication subsystem 730 may include, for example, an infrared communication device, a wireless communication device and/or chipset (such as a Bluetooth^{®} device, an IEEE 802.11 device, a Wi-Fi device, a WiMax device, cellular communication facilities, etc.), a wired communication interface, and/or similar communication interfaces. Computing system 700 may include one or more antennas 734 for wireless communication as part of wireless communication subsystem 730 or as a separate component coupled to any portion of the system. Depending on desired functionality, communication subsystem 730 may include separate transceivers to communicate with base transceiver stations and other wireless devices and access points, which may include communicating with different data networks and/or network types, such as wireless wide-area networks (WWANs), wireless local area networks (WLANs), or wireless personal area networks (WPANs). A WWAN may be, for example, a WiMax (IEEE 802.15) network. A WLAN may be, for example, an IEEE 802.11x network. A WPAN may be, for example, a Bluetooth network, an IEEE 802.6x, or some other types of network. The techniques described herein may also be used for any combination of WWAN, WLAN, and/or WPAN. Communications subsystem 730 may permit data to be exchanged with a network, other computer systems, and/or any other devices described herein. Communication subsystem 730 may include a means for transmitting or receiving data, using antenna(s) 734, wireless link(s) 732, or a wired link. Communication subsystem 730, processor(s) 710, and memory 720 may together comprise at least a part of one or more of a means for performing some functions disclosed herein.

Computing system 700 may include one or more output device(s) 760 and/or one or more input device(s) 770. Output device(s) 770 and/or input device(s) 770 may be used to provide output information and/or receive input information.

Embodiments disclosed herein may be used to implement components of an artificial reality system or may be implemented in conjunction with an artificial reality system. Artificial reality is a form of reality that has been adjusted in some manner before presentation to a user, which may include, for example, a virtual reality, an augmented reality, a mixed reality, a hybrid reality, or some combination and/or derivatives thereof. Artificial reality content may include completely generated content or generated content combined with captured (*e.g*., real-world) content. The artificial reality content may include video, audio, haptic feedback, or some combination thereof, and any of which may be presented in a single channel or in multiple channels (such as stereo video that produces a three-dimensional effect to the viewer). Additionally, in some embodiments, artificial reality may also be associated with applications, products, accessories, services, or some combination thereof, that are used to, for example, create content in an artificial reality and/or are otherwise used in (e.g., perform activities in) an artificial reality. The artificial reality system that provides the artificial reality content may be implemented on various platforms, including an HMD connected to a host computer system, a standalone HMD, a mobile device or computing system, or any other hardware platform capable of providing artificial reality content to one or more viewers.

The methods, systems, and devices discussed above are examples. Various embodiments may omit, substitute, or add various procedures or components as appropriate. For instance, in alternative configurations, the methods described may be performed in an order different from that described, and/or various stages may be added, omitted, and/or combined. Also, features described with respect to certain embodiments may be combined in various other embodiments. Different aspects and elements of the embodiments may be combined in a similar manner. Also, technology evolves and, thus, many of the elements are examples that do not limit the scope of the disclosure to those specific examples.

Specific details are given in the description to provide a thorough understanding of the embodiments. However, embodiments may be practiced without these specific details. For example, well-known circuits, processes, systems, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing various embodiments. Various changes may be made in the function and arrangement of elements without departing from the scope of the present disclosure.

Also, some embodiments were described as processes depicted as flow diagrams or block diagrams. Although each may describe the operations as a sequential process, many of the operations may be performed in parallel or concurrently. In addition, the order of the operations may be rearranged. A process may have additional steps not included in the figure. Furthermore, embodiments of the methods may be implemented by hardware, software, firmware, middleware, microcode, hardware description languages, or any combination thereof. When implemented in software, firmware, middleware, or microcode, the program code or code segments to perform the associated tasks may be stored in a computer-readable medium such as a storage medium. Processors may perform the associated tasks.

It will be apparent to those skilled in the art that substantial variations may be made in accordance with specific requirements. For example, customized or special-purpose hardware might also be used, and/or particular elements might be implemented in hardware, software (including portable software, such as applets, etc.), or both. Further, connection to other computing devices such as network input/output devices may be employed.

With reference to the appended figures, components that can include memory can include non-transitory machine-readable media. The term "machine-readable medium" and "computer-readable medium" may refer to any storage medium that participates in providing data that causes a machine to operate in a specific fashion. In embodiments provided hereinabove, various machine-readable media might be involved in providing instructions/code to processing units and/or other device(s) for execution. Additionally or alternatively, the machine-readable media might be used to store and/or carry such instructions/code. In many implementations, a computer-readable medium is a physical and/or tangible storage medium. Such a medium may take many forms, including, but not limited to, non-volatile media, volatile media, and transmission media. Common forms of computer-readable media include, for example, magnetic and/or optical media such as compact disk (CD) or digital versatile disk (DVD), punch cards, paper tape, any other physical medium with patterns of holes, a RAM, a programmable read-only memory (PROM), an erasable programmable read-only memory (EPROM), a FLASH-EPROM, any other memory chip or cartridge, a carrier wave as described hereinafter, or any other medium from which a computer can read instructions and/or code. A computer program product may include code and/or machine-executable instructions that may represent a procedure, a function, a subprogram, a program, a routine, an application (App), a subroutine, a module, a software package, a class, or any combination of instructions, data structures, or program statements.

Those of skill in the art will appreciate that information and signals used to communicate the messages described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Terms, "and" and "or" as used herein, may include a variety of meanings that are also expected to depend at least in part upon the context in which such terms are used. Typically, "or" if used to associate a list, such as A, B, or C, is intended to mean A, B, and C, here used in the inclusive sense, as well as A, B, or C, here used in the exclusive sense. In addition, the term "one or more" as used herein may be used to describe any feature, structure, or characteristic in the singular or may be used to describe some combination of features, structures, or characteristics. However, it should be noted that this is merely an illustrative example and claimed subject matter is not limited to this example. Furthermore, the term "at least one of" if used to associate a list, such as A, B, or C, can be interpreted to mean any combination of A, B, and/or C, such as A, AB, AC, BC, AA, ABC, AAB, AABBCCC, etc.

Further, while certain embodiments have been described using a particular combination of hardware and software, it should be recognized that other combinations of hardware and software are also possible. Certain embodiments may be implemented only in hardware, or only in software, or using combinations thereof. In one example, software may be implemented with a computer program product containing computer program code or instructions executable by one or more processors for performing any or all of the steps, operations, or processes described in this disclosure, where the computer program may be stored on a non-transitory computer readable medium. The various processes described herein can be implemented on the same processor or different processors in any combination.

Where devices, systems, components or modules are described as being configured to perform certain operations or functions, such configuration can be accomplished, for example, by designing electronic circuits to perform the operation, by programming programmable electronic circuits (such as microprocessors) to perform the operation such as by executing computer instructions or code, or processors or cores programmed to execute code or instructions stored on a non-transitory memory medium, or any combination thereof. Processes can communicate using a variety of techniques, including, but not limited to, conventional techniques for inter-process communications, and different pairs of processes may use different techniques, or the same pair of processes may use different techniques at different times.

The specification and drawings are, accordingly, to be regarded in an illustrative rather than a restrictive sense. It will, however, be evident that additions, subtractions, deletions, and other modifications and changes may be made thereunto without departing from the broader scope as set forth in the claims. Thus, although specific embodiments have been described, these are not intended to be limiting. Various modifications and equivalents are within the scope of the following claims.

## Claims

1. A method for utilizing contact pressure measurements of wrist-worn devices, comprising:
obtaining sensor data from one or more sensors disposed in or on a wrist-worn device worn on a wrist of a user;
determining, based on the sensor data, a measure of a contact pressure of a bottom portion of the wrist-worn device on the wrist of the user;
based on the measure of the contact pressure, providing one or more instructions to the user to adjust the wrist-worn device to bring about a change in the contact pressure toward a target contact pressure or a target contact pressure range; and
initiating a physiological measurement of the user using other sensors disposed in or on the wrist-worn device.

2. The method of claim 1, wherein identifying the target contact pressure of the wrist-worn device to the wrist of the user is based at least in part on a type of physiological measurement to be performed using another one or more sensors associated with the wrist-worn device.

3. The method of claim 1, wherein identifying the target contact pressure of the wrist-worn device to the wrist of the user is based at least in part on an activity the user is engaged in at a present time.

4. The method of claim 1, wherein identifying the target contact pressure of the wrist-worn device to the wrist of the user is based at least in part on one or more of a current time of day or current ambient temperature; and/or
wherein identifying the target contact pressure of the wrist-worn device to the wrist of the user is based at least in part on motion sensor data indicating that the user is in motion.

5. The method of claim 1, wherein providing instructions to the user to adjust the wrist-worn device to achieve the target contact pressure comprises providing instructions to adjust a band of the wrist-worn device by an amount identified based on a difference between the contact pressure determined and the target contact pressure.

6. A method for utilizing contact pressure measurements of wrist-worn devices, comprising:
obtaining sensor data from one or more sensors disposed in or on a wrist-worn device worn on a wrist of a user;
determining, based on the sensor data, a measure of a contact pressure of a bottom portion of the wrist-worn device on the wrist of the user;
identifying a target contact pressure of the wrist-worn device for a physiological measurement to be made using the wrist-worn device;
identifying at least one change to be made in connection with performing the physiological measurement based on a difference between the determined measure of the contact pressure and the target contact pressure; and
performing the physiological measurement using the at least one change to generate a physiological metric of the user.

7. The method of claim 6, wherein the at least one change comprises a change to operating characteristics of at least one component of the wrist-worn device used to perform the physiological measurement.

8. The method of claim 7, wherein the at least one component is a light source, and wherein the change to the operating characteristics comprises a change in current provided to the light source.

9. The method of claim 6, wherein the at least one change comprises a change to an analysis of sensor data collected while performing the physiological measurement to generate the physiological metric of the user.

10. The method of claim 9, wherein the change to the analysis of the sensor data comprises integrating the sensor data over a time duration that is different than a standard integration time duration, wherein the time duration is determined based at least in part on the difference between the determined contact pressure and the target contact pressure.

11. The method of claim 6, wherein the at least one change comprises a change to a manner in which the physiological metric is logged; or
wherein the at least one change comprises utilizing motion sensor data collected using one or more motion sensors of the wrist-worn device to generate the physiological metric of the user.

12. A wrist-worn device, comprising:
a top surface;
a bottom surface;
one or more force sensors; and
a processor configured to:
receive sensor data from the one or more force sensors;
determine a measure of a contact pressure of the bottom surface of the wrist-worn device to a wrist of a wearer based on the received sensor data; and
perform at least one action based on the received sensor data,
wherein the at least one action comprises at least one of: (1) causing instructions to be presented to the wearer to adjust the wrist-worn device based at least in part on the determined measure of the contact pressure; or (2) modify a manner in which a physiological measurement is made using the wrist-worn device based at least in part on the determined measure of the contact pressure.

13. The wrist-worn device of claim 12, wherein at least one force sensor of the one or more force sensors is a load sensor configured to measure a deflection of the bottom surface of the wrist-worn device; or
wherein at least one force sensor of the one or more force sensors is a strain gauge type sensor configured to measure strain.

14. The wrist-worn device of claim 133, wherein the strain gauge type sensor is disposed proximate to the bottom surface of the wrist-worn device; or
wherein the strain gauge type sensor is disposed proximate to a band connected to a portion of the bottom surface of the wrist-worn device.

15. The wrist-worn device of claim 12, wherein the at least one force sensor comprises three or more force sensors, and wherein the processor is further configured to determine a tilt of a puck of the wrist-worn device on the wrist of the wearer based on the sensor data received from the three or more force sensors, the puck comprising the top surface, the bottom surface, and a volume enclosed between the top surface and the bottom surface.
